Europäisches Patentamt

(19) European Patent Office (11) Publication number: **0 089 138**

Office européen des brevets **B1**

(12) EUROPEAN PATENT SPECIFICATION

(45) Date of publication of patent specification: **27.04.88** (51) Int. Cl.⁴: **A 61 F 5/44**

(21) Application number: **83301061.4**

(22) Date of filing: **28.02.83**

(54) A coupling for an ostomy bag.

(30) Priority: **11.03.82 GB 8207172**

(43) Date of publication of application:
**21.09.83 Bulletin 83/38**

(45) Publication of the grant of the patent:
**27.04.88 Bulletin 88/17**

(84) Designated Contracting States:
**AT BE CH DE FR IT LI LU NL SE**

(56) References cited:
**GB-A-1 571 657**
**GB-A-1 579 875**
**GB-A-1 583 027**
**US-A-4 232 672**

(73) Proprietor: **CRAIG MEDICAL PRODUCTS
LIMITED**
**Claygate House 46 Albert Road North**
**Reigate Surrey RH2 9EN (GB)**

(72) Inventor: **Steer, Peter Leslie**
**Woodlands Rise Kingscote**
**East Grinstead Sussex (GB)**
Inventor: **Edwards, John Victor**
**5, Lodge Close**
**East Grinstead Sussex (GB)**

(74) Representative: **Cook, Anthony John et al**
**D. YOUNG & CO. 10, Staple Inn**
**London, WC1V 7RD (GB)**

Courier Press, Leamington Spa, England.

## Description

This invention relates to a coupling for joining an ostomy bag or pouch to a pad or surgical dressing. Ostomy bags are usually secured to a pad or surgical dressing which contacts the user's skin and surrounds the stoma. There is a need for a coupling between pad and bag which allows the bag to be readily removed when necessary, and replaced by a clean, empty bag. At the same time, it is essential that the coupling should be a secure one, and prevent leakage particularly of liquids and gases.

One successful ostomy bag coupling invented by the present inventors, is described and claimed in British Patent Specification No. 1 571 657. While this arrangement has many advantages, it has been felt by some wearers to be desirable to have a coupling which is more flexible so that the ostomy appliance when worn follows the contours of the body more closely and so is less obtrusive under clothing and is more comfortable to wear. Other ostomy appliances are shown in British Patent Specification Nos. 1 579 875 and 1 583 027 and U.S. Patent Specification 4 232 672. With all arrangements for attaching an ostomy bag to a wearer, it is desirable to minimise the number of crevices and recesses within which faecal material can lodge or collect.

According to the invention, there is provided a coupling for joining an adhesive pad or dressing and an ostomy bag having a stomal aperture in one bag wall, the coupling comprising a body side coupling element having a lateral flange by which said body side coupling element is secured to the top surface of said adhesive pad or dressing and a bag side coupling element having a lateral flange by which said bag side coupling element is secured to the ostomy bag wall around said stomal aperture, said body side coupling element extending away from the body side lateral flange, the bag side coupling element including a continuous rib integral with the lateral flange and extending away therefrom in such a manner as to encircle the passageway through the stomal aperture in said bag wall, the outwardly extending rib of said bag coupling element having a deflectable sealing skirt extending therefrom which engages the outside of an inner wall which is integral with the lateral flange, of said body side coupling element when the coupling elements are joined, characterised in that the body side coupling element includes a series of projecting members uniformly radially spaced from said inner wall and located in an array which surrounds said inner wall, said projecting members being arcuate and having inwardly facing hook portions, in that said rib of said bag side coupling element is dimensioned to cause a temporary deformation of each of the projecting members as the coupling elements are mutually engaged and thereupon to occupy the space between said inner wall and said projecting members of said body side coupling element when the coupling elements are joined, and in that said rib includes a substantially right angle step formation arranged to engage said hook portion of said arcuate projecting members when the coupling elements are joined.

In conjunction with the hook portion on each of the projections, the right angle step formation provides improved security of attachment of the two coupling elements.

According to an optional feature of the invention, the bag side coupling element may be formed to define a gas vent structure including a recess for receiving a filter member or fitted pad. The filter member or pad may consist of or carry material such as activated carbon which will remove odorous gases from any flatus gases which are vented from the bag.

The array of projections may for example be made up of a plurality of arcuate ribs, e.g. 12 in number, each of which is spaced from its neighbor by a space whose length measured around the periphery is from 1/10 to 1/2 of the peripheral length of each arcuate rib. Each arcuate rib may have a hook portion defined by a smoothly curved surface and a straight hooking surface, as seen in vertical axial cross-section. The hooking surface may be at an angle from 50 to 70°, preferably 60°, to the axis of the coupling.

Fig. 1 is a front view of a first or body side coupling element of a coupling according to one embodiment of the invention not showing the adhesive pad or dressing;

Fig. 2 is a vertical axial cross-section through the coupling element shown in Fig. 1 but showing the adhesive pad or dressing;

Fig. 3 is a vertical axial section on an enlarged scale, compared to Fig. 2, showing the detail of the construction of the first coupling element;

Fig. 4 is a rear view of one example of a second or bag side coupling element, not showing the bag, intended to cooperate with the body side coupling element of Figs. 1—3;

Fig. 5 is a vertical axial cross-section, on an enlarged scale, showing the detailed construction of the coupling element of Fig. 4 as attached to an ostomy bag;

Fig. 6 is a vertical axial cross-section through an upper portion of the element of Fig. 4, showing a recess for receiving a filter member or a filter pad;

Fig. 7 is a rear view of the gas vent structure including the recess shown in Fig. 6; and

Fig. 8 is a vertical axial cross-section of the coupling elements attached.

The illustrated first and second coupling elements (24, 24A) are of closed loop form and define apertures 20, 22 intended to receive the stoma of the wearer. While these apertures have been illustrated as circular, there is no reason why they should not be elliptical or of other shape; however, a circular configuration has been found to be satisfactory. These coupling elements may each be integrally-molded from a moldable synthetic plastics material. One example of a suitable material is that known as EVA, i.e., polymerized ethylene vinyl acetate. Other synthetic plastics materials may be suitable.

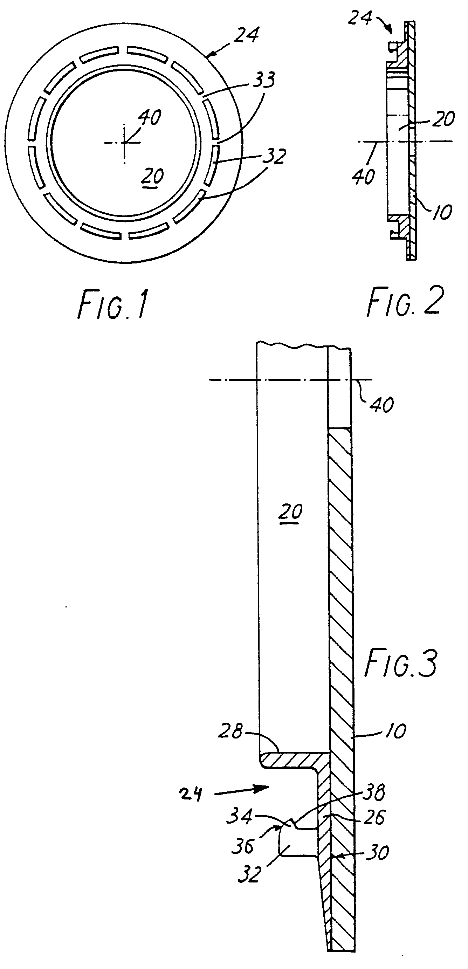

The first coupling element 24 has a lateral flange 26 and an inner wall 28. The surface 30 of the flange is intended to be secured, for example, by adhesive, to the surface of an adhesive pad or dressing 10 as shown in Figs. 2, 3 and 8. The adhesive pad or dressing can be provided with an aperture similar, though of smaller diameter, than aperture 20. Alternatively, the pad or dressing can be provided with a small starter hole that the ostomate increases in size to fit snuggly around the stoma.

The adhesive pad or dressing 10 can be made from any of the materials currently employed for this purpose. Suitable adhesive dressings are taught in U.S. Patents 3,339,546 and 3,972,328 and in U.S. Patent 4,192,785.

The inner wall 28 completely encircles the stoma aperture 20, and a series of projecting members 32 are secured to or integral with the flange 26, spaced from one another in a peripheral direction, and radially spaced from the inner wall 28. In other words, these projecting members are disposed in a circular array which surrounds the inner wall 28. In conjunction with the inner wall 28 they define a space which, when the coupling is connected, is occupied by a rib member to be described.

Each projecting member 32 is arcuate as can be seen from Fig. 1 and each has an inwardly facing hook portion 34. This may extend along the whole peripheral length of each arcuate projection 32. As best seen in Fig. 3, each arcuate projection 32 may have its hook portion defined by smoothly curved surface 36 and a straight hooking surface 38. As seen in vertical axial cross-section, the straight surface 38 may be disposed at an angle of 60° to the axis 40 of the coupling.

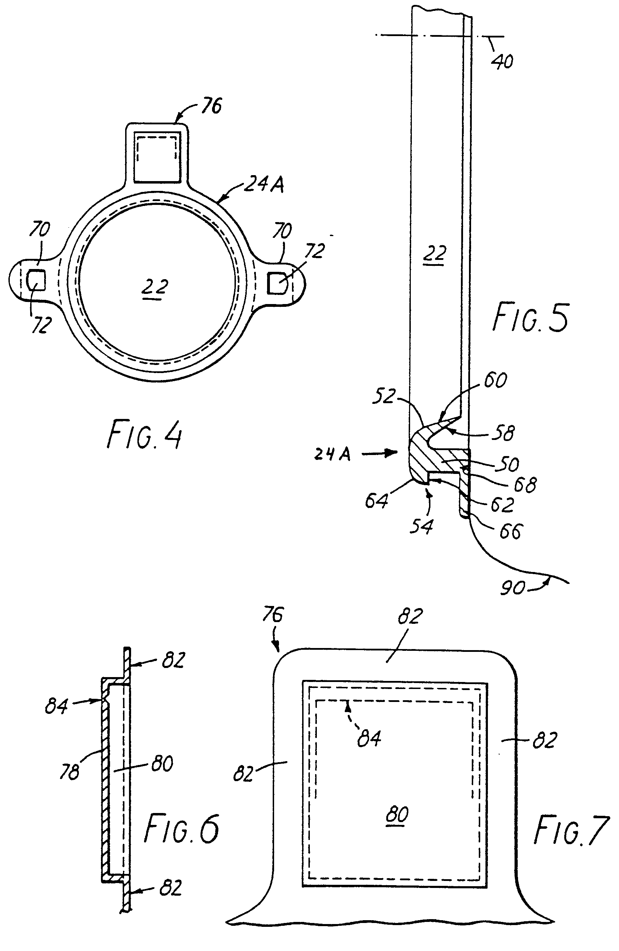

The second or bag side coupling element 24A is intended to cooperate with the body side coupling element 24 just described. The second coupling element is shown in Figs. 4—7 and includes rib member 50 encircling the aperture 22, the rib member 50 being integral with a deflectable sealing skirt 52 located on the radially inner side of the rib 50 and with a step formation 54 located on the radially outer side of the rib 50. The deflectable sealing skirt or sealing strip 52 may take the form described in British Patent 1,586,823. As seen in Fig. 5, it has two surfaces 58, 60 which taper towards one another, the radially outer surface 58 being disposed preferably at 60° to the axis 40 of the coupling and the radially inner surface 60 being disposed preferably at 30° to the said axis 40. It has been found that the illustrated configuration gives a good compromise between the conflicting requirements of tight liquid-proof sealing and ease of separation of coupling manually by old, infirm, or not particularly dextrous persons. The step formation 54 is in part defined by a surface 62 which is located in a plane substantially normal to the axis 40, although of course a surface located in a plane at a small angle to the normal could also be employed. The surface 62 in combination with the curved surface 64 defines a substantially right angle corner which, when the two coupling elements are joined together, engages sloping hook surface 38 on the first coupling element. The resilience of the plastics coupling elements and the curving of surfaces 36 and 64 lead to a construction in which the two coupling elements can be readily pushed together into their locked condition, each coupling element deforming slightly as it is engaged with the other one and then springing back to the normal configuration illustrated in Figs. 3 and 5 respectively. The provision of spaces 33, Fig. 1, between the individual projections 32 means that this deformation is readily achieved without needing to apply a substantial force.

The second coupling element also includes a lateral flange 66 whose surface 68 is secured in any convenient manner, for example, by adhesive, to one wall of an ostomy bag 90. This is preferably done during the manufacture of the ostomy bag and a plastics heat welding operation may be employed. The ostomy bag 90 may be a closed bag as shown, for example, in U.S. Patent 3,302,647 or a bag having a drainable resealable bottom opening as shown, for example, in U.S. Patent 3,612,053.

Fig. 8 shows the body side coupling of Figs. 1 to 3 and the bag side coupling of Figs. 4 and 5 without optional gas vent structure 76 in their coupled state.

As seen best in Fig. 4, the second coupling element 24A has gripping tags 70, each having a hole 72 therein so that a belt or strap may, if desired, be attached to the coupling element 24A to support the weight of the bag.

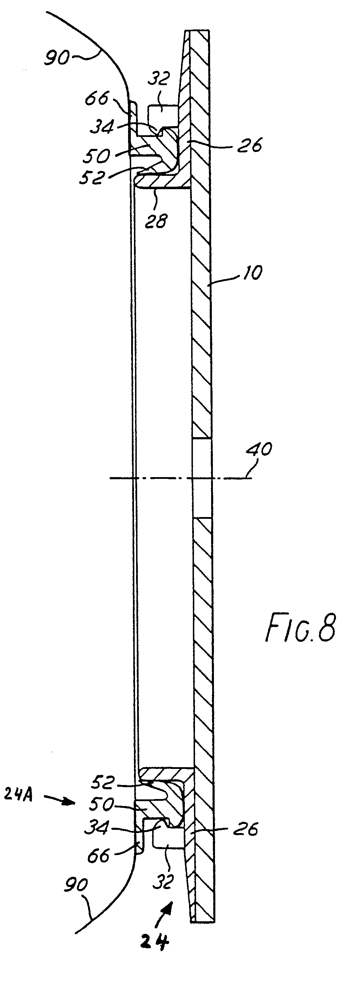

The second coupling element 24A is also integrally formed, according to an optional embodiment of the invention, with an integral gas vent structure 76 consisting of a plastics wall 78 defining a recess 80 (Figs. 6 and 7). The recess 80 is intended to receive a filter member or a filter pad; for example one may employ open-cell polyurethane foam loaded with activated carbon particles. The wall 78 of the recess has a flat surface 82 which is secured to the ostomy bag wall during the manufacture of the ostomy bag or during the attachment of the coupling to the ostomy bag, and a line of weakening 84 is disposed across the wall 78 as indicated. The line of weakening 84 is "goal-post" shape and when finger pressure is applied the wall 78 breaks along the line 84 and so provides an exit path for gases which have passed through the filter pad, not shown, located in the recess 80. The ostomy bag wall adjacent the second coupling element is punctured in a region which is within the boundary defined by the surface 82, so affording a path for flatus gases within the bag to pass out of the hole so punctured and into the filter pad located in the recess 80.

With this construction, the user may use the gas vent filtering feature if he wishes, but need not do so. Moreover, this construction allows the filter pad to be readily removed and replaced if desired.

This invention also extends to a first coupling element having an inner wall continuously encircling an aperture and also having a series of projecting members located in an array which surrounds the inner wall, peripherally spaced from one another, the inner wall and the projecting members being connected to a lateral flange.

The invention further provides a coupling element which includes a rib member encircling an aperture and which is constructed to make sealing engagement by way of a sealing skirt with a facing surface, the rib having on its radially outer surface a step formation of substantially right angle configuration and located such that it can effect a locking action with a confronting angled surface.

The ostomy bag coupling particularly described and illustrated herein has the following useful and advantageous improved features, compared to any prior coupling known to the applicants. Firstly, it has more flexibility, that is to say the coupling when worn by a wearer can deform more readily to follow the curvature of the body as the wearer moves, bends, and twists. This extra flexibility is achieved principally by the presence of separate spaced projections separated by spaces or slots, compared to the more rigid construction which is a consequence of a peripheral channel arrangement as illustrated in British Patent No. 1,571,657.

Secondly, the smooth clean configuration of the interior surface of the coupling, that is to say the interior surface of the wall 28 on the first coupling element, means that there is less likelihood for unwanted deposits, e.g., of faeces, to build up. Moreover, the action of removing the bag side coupling element from the body side coupling element involves a wiping action which usually wipes clean the radially outer surface of the wall 28 and the inwardly facing surfaces of the projections 32. In consequence, build-up of unwanted matter within the space between the wall 28 and the projections 32 is less likely to occur.

Thirdly, by the employment of the dimensions and proportions indicated in Figs. 3 and 5, a coupling having a lesser "outstand" may be produced. That is to say, the extent of projection of the coupling away from the body of the wearer is less, which is an advantage in that it makes the coupling and ostomy bag less likely to be visible or noticed under the clothing of the wearer. Moreover, it is less cumbersome to wear. With a coupling according to Figs. 3 and 5, a reduction of over 30% in overall thickness or "outstand" has been made, compared to a widely-used prior known coupling.

Fourthly, the construction disclosed and illustrated herein allows the incorporation of a gas vent structure and a gas filter as part of the bag side coupling and therefore no special extra filter arrangement or gas vent structure is needed.

Fifthly, the security of attachment of the two coupling elements is enhanced by the choice of the particular engaging configuration, namely the right angle step portion cooperating with a confronting angled surface of the hook portions on the array of projections.

## Claims

1. A coupling for joining an adhesive pad or dressing (10) and an ostomy bag (90) having a stomal aperture (22) in one bag wall, the coupling comprising a body side coupling element (24) having a lateral flange (26) by which said body side coupling element (24) is secured to the top surface of said adhesive pad or dressing (10) and a bag side coupling element (24A) having a lateral flange (66) by which said bag side coupling element (24A) is secured to the ostomy bag wall around said stomal aperture (22), said body side coupling element (24) extending away from the body side lateral flange (26), the bag side coupling element (24A) including a continuous rib (50) integral with the lateral flange (66) and extending away therefrom in such a manner as to encircle the passageway through the stomal aperture (22) in said bag wall, the outwardly extending rib (50) of said bag coupling element (24A) having a deflectable sealing skirt (52) extending therefrom which engages the outside of an inner wall (28) which is integral with the lateral flange (26) of said body side coupling element (24) when the coupling elements (24, 24A) are joined, characterised in that the body side coupling element (24) includes a series of projecting members (32) uniformly radially spaced from said inner wall (28) and located in an array which surrounds said inner wall, said projecting members (32) being arcuate and having inwardly facing hook portions (34), in that said rib (50) of said bag side coupling element (24A) is dimensioned to cause a temporary deformation of each of the projecting members (32) as the coupling elements (24, 24A) are mutually engaged and thereupon to occupy the space between said inner wall (28) and said projecting members (32) of said body side coupling element (24) when the coupling elements are joined, and in that said rib (50) includes a substantially right angle step formation (62) arranged to engage said hook portion (34) of said arcuate projecting members (32) when the coupling elements (24, 24A) are joined.

2. A coupling according to Claim 1 wherein each arcuate projecting member (32) has a hook portion (34) defined by a smoothly curved surface and a straight hooking surface (38) when seen in vertical cross-section.

3. A coupling according to Claim 2 wherein said hooking surface (38) is at an angle from about 50° to about 70° to the axis of the coupling.

4. A coupling according to Claim 1, 2 or 3 wherein said adhesive pad or dressing (10) has an aperture which can be enlarged to fit snugly around the stoma and the lateral flange (26) of said body side coupling element (24) is secured to the surface of said pad or dressing such that said inner wall (28) encircles the passageway (20) through which the stoma will protrude.

## Patentansprüche

1. Kupplung zum Verbinden eines Haftkissens oder einer Unterlage (10) mit einem Ostomiebeutel (90), der eine Stomaöffnung (22) in einer Beutelwand hat, wobei die Kupplung ein körperseitiges Kupplungselement (24) aufweist, das einen Querflansch (26) hat, durch den dieses körperseitige Kupplungselement (24) fest mit der Oberfläche des Haftkissens oder der Unterlage (10) verbunden ist, und ein beutelseitiges Kupplungselement (24A) aufweist, das einen Querflansch (26) hat, durch den das beutelseitige Kupplungselement (24A) fest mit der Ostomiebeutelwand um die Stomaöffnung (22) verbunden ist, wobei das körperseitige Kupplungselement (24) von dem körperseitigen Querflansch (26) sich weg erstreckt, das beutelseitige Kupplungselement (24A) eine durchgehende Rippe (50) aufweist, die einteilig mit dem Querflansch (26) ausgebildet ist und sich von diesem derart weg erstreckt, daß der Durchgang durch die Stomaöffnung (22) in der Beutelwand umgeben wird, wobei die nach außen verlaufende Rippe (50) des Beutelkupplungselements (24A) einen biegsamen, sich hiervon weg erstreckenden Dichtrand (52) hat, der in Eingriff mit der Außenseite einer inneren Wand (28) ist, die einteilig mit dem Querflansch (26) des körperseitigen Kupplungselements (24) verbunden ist, wenn die Kupplungselemente (24, 24A) verbunden sind, dadurch gekennzeichnet, daß das körperseitige Kupplungselement (24) eine Reihe von vorspringenden Teilen (32) enthält, die gleichförmig radial im Abstand von der inneren Wand (28) angeordnet und in einer Gruppe vorgesehen sind, die die innere Wand umgibt, daß die vorspringenden Teile (32) gewölbt sind und nach innen weisende Hakenabschnitte (34) haben, daß die Rippe (50) des beutelseitigen Kupplungselements (24A) derart bemessen ist, daß eine temporäre Verformung jedes der vorspringenden Teile (32) bewirkt wird, wenn die Kupplungselemente (24, 24A) wechselseitig in Eingriff sind und daß sie dann den Raum zwischen der inneren Wand (28) und den vorspringenden Teilen (32) des körperseitigen Kupplungselements (24) einnehmen, wenn die Kupplungselemente verbunden sind, und daß die Rippe (50) eine im wesentlichen rechtwinklige Stufenausbildung (62) enthält, die derart angeordnet ist, daß ein Eingriff mit dem Hakenabschnitt (34) der gewölbten vorspringenden Teile (32) vorhanden ist, wenn die Kupplungselemente (24, 24A) verbunden sind.

2. Kupplung nach Anspruch 1, bei der jedes gewölbte, vorspringende Teil (32) einen Hakenabschnitt (34) hat, der von einer gleichmäßig gekrümmten Fläche und eine gerade Hakenfläche (38) in einer Vertikalschnittansicht gesehen begrenzt wird.

3. Kupplung nach Anspruch 2, bei der die Hakenfläche (38) einen Winkel von etwa 50° bis etwa 70° zur Achse der Kupplung hat.

4. Kupplung nach Anspruch 1, 2 oder 3, bei der das Haftkissen oder die Unterlage (10) eine Öffnung hat, die vergrößert werden kann, um sich eng sitzend um das Stoma anzuschmiegen, und daß der Querflansch (26) des körperseitigen Kupplungselements (24) fest mit der Fläche des Kissens oder der Unterlage derart verbunden ist, daß die innere Wand (28) den Durchgang (20) umgibt, durch die das Stoma ragt.

## Revendications

1. Raccord permettant de joindre un tampon ou pansement adhésif (10) et une poche de stomie (90) comportant une ouverture stomiale (22) dans une paroi de la poche, ce raccord comprenant un élément de raccord côté corps (24) comportant un rebord latéral (26) par lequel ledit élément de raccord côté corps (24) est fixé à la surface supérieure dudit tampon ou pansement adhésif (10), et un élément de raccord côté poche (24A) comportant un rebord latéral (66) par lequel ledit élément de raccord côté poche (24A) est fixé à la paroi de la poche de stomie autour de ladite ouverture stomiale (22), ledit élément de raccord côté corps (24) dépassant du rebord latéral (26) côté corps, l'élément de raccord côté poche (24A) comportant une nervure continue (50) qui fait corps avec le rebord latéral (66) et qui en dépasse de manière à encercler le passage qui traverse l'ouverture stomiale (22) dans ladite paroi de la poche, la nervure (50), dirigée vers l'extérieur, dudit élément de raccord côté poche (24A) comportant une jupe d'étanchéité flexible (52) qui en part et qui s'engage sur la face extérieure d'une paroi intérieure (28) faisant corps avec le rebord latéral (26) dudit élément de raccord côté corps (24) quand les éléments de raccord (24, 24A) sont réunis, caractérisé en ce que l'élément de raccord côté corps (24) comporte une série de saillies (32) espacées radialement, de manière uniforme, de ladite paroi intérieure (28) et formant une rangée qui entoure ladite paroi intérieure, lesdites saillies (32) étant en forme d'arc de cercle et ayant des parties (34) en forme de crochet tournées vers l'intérieur, en ce que ladite nervure (50) dudit élément de raccord côté poche (24A) a des dimensions propres à provoquer une déformation temporaire de chacune des saillies (32) tandis que les éléments de raccord (24, 24A) sont en prise mutuelle, puis à occuper l'espace situé entre ladite paroi intérieure (28) et lesdites saillies (32) dudit élément de raccord côté corps (24) quand les éléments de raccord sont réunis, et en ce que ladite nervure (50) comporte un gradin (62) sensiblement à angle droit, disposé de façon à venir en prise avec ladite partie en forme de crochet (34) desdites saillies en forme d'arc de cercle (32) quand les éléments de raccord (24, 24A) sont réunis.

2. Raccord selon la revendication 1, dans lequel chaque saillie en forme d'arc de cercle (32) comporte une partie en forme de crochet (34) délimitée par une surface doucement incurvée et par une surface d'accrochage rectiligne (38), vues en coupe verticale.

3. Raccord selon la revendication 2, dans lequel ladite surface d'accrochage (38) forme un angle d'environ 50° à environ 70° avec l'axe du raccord.

4. Raccord selon la revendication 1, 2 ou 3, dans lequel ledit tampon ou pansement adhésif (10) comporte une ouverture que l'on peut agrandir pour qu'elle s'adapte étroitement autour de l'ouverture stomiale, et en ce que le rebord latéral (26) dudit élément de raccord côté corps (24) est fixé à la surface dudit tampon ou pansement de telle sorte que ladite paroi intérieure (28) fait le tour du passage (20) par lequel l'ouverture stomiale fera saillie.

*FIG.1*

*FIG. 2*

*FIG.3*

0 089 138

FIG.4

FIG.5

FIG.6

FIG.7

FIG.8